# FASCICULE DE BREVET EUROPEEN

(11) **EP 1 173 134 B1**
(45) Date de publication et mention de la délivrance du brevet: **11.05.2011**
(21) Numéro de dépôt: 00926770.9
(22) Date de dépôt: 29.03.2000
(51) Int. Cl.: A61J 1/00, B32B 27/00

(54) **STRUCTURE THERMOPLASTIQUE MULTICOUCHE**
THERMOPLASTISCHE MEHRSCHICHTIGE STRUKTUR
MULTILAYER THERMOPLASTIC STRUCTURE

(30) Priorité: 08.04.1999 BE 9900244
(43) Date de publication de la demande: 23.01.2002
(73) Titulaire: RENOLIT Nederland B.V., 1601 MA Enkhuizen (NL)
(72) Inventeur: KARSTEN, Petrus, J., A., NL-1602 DL Enkhuizen (NL)
(74) Mandataire: Wagner, Jutta
(86) Numéro de dépôt international: PCT/EP2000/002759
(87) Numéro de publication internationale: WO 2000/061062

(56) Documents cités:
- US-A- 4 775 562
- US-A- 4 828 892
- US-A- 4 929 479
- ASANUMA T.; NAKANISHI S.; SHIOMURA T.: KANAYA T.: 'Crystallization of Syndiotactic Polypropylene' SEN-1 GAKKAISHI vol. 49, no. 5, 1993, pages 260 - 263

## Description

La présente invention concerne une structure multicouche à base de matière thermoplastique, telle qu'un tube ou un film, utilisable notamment pour la fabrication d'articles pour applications médicales.

Les articles destinés à des applications médicales doivent répondre non seulement à des exigences classiques telles qu'une bonne résistance mécanique ou un faible coût, mais également à des exigences - extrêmement strictes- propres à ce domaine d'application spécifique, telles que par exemple des exigences concernant les propriétés de biocompatibilité desdits articles, leur aptitude à subir un traitement de stérilisation, leur flexibilité, leur transparence, leur soudabilité, leur résistance au choc (y compris en ce qui concerne les récipients remplis de liquide), ou la quantité de substances extractibles (par exemple à l'hexane).

Jusqu'à présent, les articles à usage médical disponibles commercialement, par exemple les poches à perfusion ou à sang, sont à base de polymères du chlorure de vinyle, par exemple de PVC. Bien que présentant plusieurs avantages, ce type de polymère présente cependant certains inconvénients, tels que la nécessité de lui incorporer des quantités élevées de stabilisants en vue d'améliorer sa stabilité thermique, de lui incorporer des quantités élevées de plastifiants en vue d'obtenir une souplesse suffisante. Le marché est donc demandeur d'articles pour applications médicales exempts de polymères chlorés.

Des articles de ce type, à base de polyoléfines, ont déjà été proposés. Ainsi, par exemple, la demande internationale de brevet WO 97/34951 (Sengewald) décrit des films multicouches à base de polyoléfines, utilisables notamment dans des applications médicales.

D'autre part, les articles connus à base de copolymères présentent cependant l'inconvénient fréquent de comprendre une quantité notable de copolymère(s) à teneur élevée en comonomère, ce qui accroît certes leur flexibilité mais réduit leur température de fusion, ce qui est désavantageux quant à la stabilité dimensionnelle des articles lors d'une stérilisation à la vapeur (121 °C), et conduit souvent à une teneur élevée en constituants extractibles. En outre, la transparence de ces articles est souvent médiocre, en raison de la cristallisation rapide des polyoléfines généralement utilisées.

Par ailleurs, les articles connus à base d'homopolymères du propylène comprennent souvent des polymères de faible isotacticité, ce qui permet certes d'obtenir une bonne souplesse, mais conduit à une teneur élevée en constituants extractibles. En outre, ils présentent souvent une npolydispersité élevée, ce qui signifie que leurs plages de températures de fusion et de cristallisation se recouvrent largement, ce qui rend impossible une réduction efficace de la cristallinité et l'obtention d'une bonne transparence.

En vue de remédier à ces inconvénients, la présente invention concerne une structure multicouche à base de polymères thermoplastiques, substantiellement exempte de polymères du chlorure de vinyle et de plastifiants de faible masse moléculaire, comprenant au moins 3 couches:
- une première couche (A) comprenant au moins 60 % en poids d'au moins une polyoléfine à cristallinité contrôlable, l'épaisseur de la première couche étant d'au moins 20 % de l'épaisseur totale de la structure, la première couche ayant un module d'élasticité inférieur à 350 MPa;
- une seconde couche (B), disposée entre la première (A) et la troisième (C) couche, comprenant au moins 40 % en poids d'au moins une polyoléfine à cristallinité contrôlable, la seconde couche ayant globalement une température de ramollissement inférieure à 121 °C;
- une troisième couche (C) comprenant au moins 50 % en poids d'au moins une polyoléfine à cristallinité contrôlable, l'épaisseur de la troisième couche étant de 5 à 30 % de l'épaisseur totale de la structure, et la troisième couche ayant un module d'élasticité inférieur à celui de la première couche, dans laquelle la polyoléfine à cristallinité contrôlable est définie comme comprenant au moins 90% de propylène et comme avant une température de ramollissement inférieure à 121 °C.

Par structure multicouche, on entend désigner tout produit semi-fini multicouche, et plus particulièrement tout film, feuille, tube ou récipient.

Un important constituant de la structure multicouche de l'invention, présent dans chacune des trois couches A, B et C, est une polyoléfine à cristallinité contrôlable. Cette expression désigne, dans le contexte de la présente invention, une polyoléfine comprenant au moins 90 %, de propylène et ayant une température de ramollissement (un "point Vicat") inférieure à 121 °C (dans la présente demande, toutes les températures de ramollissement sont mesurées selon la norme ASTM D 1525). La température de ramollissement dont il est question se rapporte à la polyoléfine à cristallinité contrôlable telle qu'elle est présente dans la structure de l'invention. En particulier, il est possible d'utiliser une polyoléfine dont la température de ramollissement indiquée par le fabricant est supérieure à 121 °C, en choisissant les conditions de fabrication de la structure de façon telle que la température de ramollissement réelle de ladite polyoléfine au sein de ladite structure soit inférieure à 121 °C. Les polyoléfines de ce type présentent la particularité que leur cristallinité peut aisément être réduite au cours de leur mise en oeuvre. Avantageusement, les "polyoléfines à cristallinité contrôlable" présentent un temps de relaxation τ₀ d'au moins 10 s (et de préférence d'au moins 15 s) à la température d'extrusion (T₀) et en l'absence de toute contrainte d'étirage. Ce temps de relaxation τ₀ est défini par la relation dans laquelle η₀ désigne la viscosité dynamique de la polyoléfine pour des gradients de vitesse tendant vers 0, p est sa masse volumique, Mc est la masse moléculaire (moyenne en poids) critique au-delà de laquelle η₀ est proportionnelle à la puissance 3,4 de la masse moléculaire, R est la constante des gaz parfaits, et T₀ est égal à Tf - 20 °C, Tf désignant la température de fusion. Ce contrôle aisé de la cristallinité est avantageux dans le contexte de l'invention, dans la mesure où il permet de conférer à ces polyoléfines, pour autant que l'on utilise des conditions de mise en oeuvre appropriées, une cristallinité réduite, accroissant ainsi la transparence et la souplesse de l'ensemble de la structure. C'est pour cette raison que ces polyoléfines sont qualifiées de "polyoléfines à cristallinité contrôlable".

Plusieurs telles polyoléfines peuvent être utilisées en mélange, que ce soit dans l'une ou plusieurs des couches A, B et C, la quantité minimale indiquée ci dessus concernant dans ce cas la somme des quantités de chacune de ces polyoléfines. Les polyoléfines à cristallinité contrôlable présentes dans les couches A, B et C peuvent être identiques ou différentes.

Cette polyoléfine à cristallinité contrôlable peut être un homopolymère , du propylène. A titre d'exemples non-limitatifs de tels homopolymères, on peut citer les homopolymères du propylène (PP) [y compris les PP syndiotactiques (s-PP), stéréo-blocs (s-b-PP) ou iso-blocs PP (i-b-PP)]. Les homopolymères préférés sont les polypropylènes syndiotactiques, stéréo-blocs et iso-blocs.

On préfère que la polyoléfine à cristallinité contrôlable soit un copolymère. Il peut notamment s'agir d'un copolymère de plusieurs de ces trois monomères (éthylène, propylène, butène), comprenant au moins 90 % du propylène et moins de 10 % en tout de l'un ou de chacun des deux autres monomères. Selon une autre variante, la polyoléfine à cristallinité contrôlable est un copolymère comprenant au moins 90 % en poids, de propylène, ainsi que moins de 10 % d'un ou plusieurs autres comonomères choisis dans le groupe constitué d'hydrocarbures en C5 à C10 (exemples : pentène-1,hexène-1, octène-1, 4-methylpentène-1, cyclohexène, norbornène,...).

On notera que dans la présente demande, en l'absence d'indications contraires, le terme "copolymères" englobe les polymères comprenant deux comonomères ou davantage.

En ce qui concerne les copolymères, la quantité du ou des comonomères minoritaires est de préférence d'au moins 1,5 % en poids. On préfère en particulier utiliser comme polyoléfine à cristallinité contrôlable un copolymère comprenant au moins 90 % en poids de propylène et au moins 2 % en poids d'éthylène et/ou de butène.

En ce qui concerne les homopolymères et les copolymères, leur polydispersité est avantageusement inférieure à 8, et de préférence inférieure à 4. Cette caractéristique traduit une faible dispersion des masses moléculaires, qui accroît l'écart entre les plages de températures de fusion et de ramollissement, et permet ainsi d'agir plus efficacement sur la cristallinité au cours de la mise en oeuvre.

Par ailleurs, l'indice de fluidité des polyoléfines à cristallinité contrôlable utilisées est avantageusement inférieur à 10 g/10 min, de préférence inférieur à 5 g/10 min (mesuré selon ASTM D1238, dans les conditions190 °C/2,16 kg pour les polymères de l'éthylène et les copolymères du butène, et dans les conditions 230 °C/2,16 kg pour les polymères du propylène et les homopolymères du butène).

De préférence, au moins une polyoléfine à cristallinité contrôlable comprise dans la première couche (A) présente une température de fusion supérieure à 121 °C, et plus préférentiellement supérieure à 130 °C. Avantageusement, au moins une polyoléfine à cristallinité contrôlable comprise dans la troisième couche (C) présente une température de fusion supérieure à 121 °C, et plus préférentiellement supérieure à 130 °C. On préfère qu'il en aille de même pour au moins une polyoléfine à cristallinité contrôlable comprise dans la seconde couche (B). Cette caractéristique est intéressante en vue d'une stérilisation à la vapeur (à 121 °C).

Outre une ou plusieurs polyoléfines à cristallinité contrôlable telles que décrites ci-dessus, les couches A, B et C peuvent éventuellement contenir un ou plusieurs autres polymères thermoplastiques. Avantageusement, on choisit ces autres polymères parmi les polyoléfines peu cristallines ou amorphes, des types suivants :
- les copolymères oléfiniques constitués d'au moins deux alcènes en C2 à C10, comprenant au moins 60 % en poids d'éthylène et/ou de propylène et/ou de butène, mais ne comprenant pas plus de 90 % en poids d'un même comonomère, ou parmi
- les copolymères oléfiniques comprenant de l'éthylène et/ou du propylène et/ou du butène ainsi que de 10 à 40 % en poids d'un ou plusieurs comonomères différents (qui sont de préférence choisis parmi les oléfines en C5 à C10 et les groupements acides ou esters carboxyliques, par exemple acétate de vinyle, acrylate de méthyle, d'éthyle ou de butyle ainsi que méthacrylate de méthyle, ou encore monoxyde de carbone), ou parmi
- les copolymères élastomériques à blocs du styrène et d'une oléfine (par ex. des copolymères du type styrène-butadiène-styrène, ou styrène-éthylène-propylène-styrène, etc.), ou encore parmi
- les homopolymères fortement branchés [par exemple le polyéthylène de densité faible (LDPE) ou moyenne (MDPE)] et enfin parmi
- les copolymères d' oléefines cycliques (COC), tels que les copolymères à base de norbornène par exemple.

Par COC, on entend des copolymères à base d'une oléfine en C2 à C10 et d'un monomère cyclique en C5 à C16, dans une teneur respective de 20 à 98% en poids pour l'oléfine et de 2 à 80% en poids pour le monomère cyclique. L'oléfine est avantageusement l'éthylène. Le monomère cyclique peut être le cyclopentène, le cyclohexène, le dicyclopentadiène, le tétracyclododécène ou le méthyltétracyclododécène. Le COC est de préférence un copolymère d'éthylène et de norbomène. Dans ce cas, la teneur en éthylène est avantageusement comprise entre 30 et 80 % en poids et celle en norbornène, entre 20 et 70 %.

Des exemples de polyoléfines peu cristallines ou amorphes utilisables sont les copolymères éthylène-acétate de vinyle (EVA), éthylène-acrylate de méthyle (EMA), éthylène-acrylate d'éthyle (EEA), éthylène-acrylate de butyle (EBA), les copolymères de l'éthylène de faible densité (LLDPE, VLDPE,ULDPE), les élastomères polyoléfiniques (POE) et les copolymères d'oléofines cycliques (COC). Parmi les copolymères cités ci-dessus, seuls entrent naturellement en considération comme constituants additionnels, ceux qui présentent une teneur en comonomère(s) d'au moins 10%.

Selon une variante préférée, le ou les polymères constitutifs de la couche A sont exclusivement choisis parmi le groupe constitué des polyoléfines à cristallinité contrôlable et des polyoléfines peu cristallines ou amorphes telles que décrites ci-dessus. Cette variante avantageuse concerne également les couches B et C, indépendamment l'une de l'autre et indépendamment de la couche A.

Selon des variantes avantageuses de l'invention, les couches A, B et C présentent des propriétés plus particulières, décrites ci-dessous.

Selon une variante avantageuse, la couche A présente globalement une température de fusion supérieure à 121 °C. De préférence, au moins une polyoléfine à cristallinité contrôlable comprise dans la couche A présente un indice de fluidité (mesuré dans les conditions mentionnées ci-dessus) inférieur à 10 g/10 min. Le module d'élasticité de la couche A n'excède pas 350 MPa (dans la présente demande, tous les modules d'élasticité sont mesurés selon la norme ASTM D882).

Selon une variante avantageuse, la couche B présente globalement une température de fusion supérieure à 121 °C. De préférence, son module d'élasticité est inférieur à 275 MPa, et plus particulièrement à 150 MPa. On préfère par ailleurs que l'épaisseur de la couche B soit de 20 à 70 % de l'épaisseur totale de la structure.

Selon une variante avantageuse, la couche C présente globalement une température de fusion supérieure à 121 °C. Il est cependant avantageux, tout en respectant cette condition, que la couche C comprenne au moins un polymère dont la température de fusion soit inférieure à 121 °C, ceci afin d'améliorer la soudabilité de la structure. La quantité de ce(s) polymère(s) est de préférence de 1 à 10 % du poids de la couche C, voire de 2.5 à 7.5%. Ce ou ces polymères peuvent être choisis parmi les polyoléfines à cristallinité contrôlable et les polyoléfines peu cristallines ou amorphes. Il s'agit de préférence de copolymères éthylène-acétate de vinyle (EVA), éthylène-acrylate de méthyle (EMA), éthylène-acrylate d'éthyle (EEA), éthylène-acrylate de butyle (EBA), les copolymères de l'éthylène de faible densité (LLDPE, VLDPE, ULDPE). Ces polymères présentent de préférence un température de fusion de 70 à 105°C environ. On choisit avantageusement ces polymères de telle sorte qu'ils ne sont que peu ou pas miscibles avec les autres ingrédients de la couche C. Par ailleurs, de préférence, le module d'élasticité de la couche C est inférieur à 275 MPa.

Selon une variante préférée, la couche A présente un module d'élasticité supérieur à celui de la couche B et à celui de la couche C. Selon une variante avantageuse, la couche A présente une température de ramollissement supérieure à celle de la couche B et à celle de la couche C. Selon une variante préférée, la couche A présente une température de fusion supérieure à celle de la couche B et à celle de la couche C.

De préférence, la teneur en aluminium extractible de la structure est inférieure à 1 ppm (selon la Pharmacopée Européenne).

Il s'est révélé avantageux que la structure présente globalement une masse spécifique inférieure à 0,905 kg/dm³. En outre, on préfère utiliser les variantes dans lesquelles la température de fragilisation (telle que définie par la norme DIN 53372) est d'au plus 4 °C.

Dans une variante avantageuse, le rapport [contrainte à la rupture / contrainte au seuil d'écoulement] (en anglais : Tensile strength/Yield strength) est supérieur à 1,5 (mesuré selon la norme ASTM D882), et de préférence supérieur à 2.

Les plastifiants de faible masse moléculaire (Mw < 1000) sont substantiellement exclus de la structure multicouche de l'invention. Ceci concerne en particulier les plastifiants monomériques, tels que par exemple ceux à base de phtalates. La caractéristique selon laquelle la structure de l'invention est "substantiellement exempte" de polymères du chlorure de vinyle et de plastifiants de faible masse moléculaire signifie que d'éventuels constituants de ce type, s'ils sont présents, le sont en des proportions n'excédant pas 0,1 % en poids. De préférence, ces composés sont totalement absents de la structure.

La structure multicouche comprend au moins les trois couches A/B/C mentionnées ci-dessus, dans cet ordre, ainsi qu'éventuellement une ou plusieurs autres couches de matière thermoplastique. Le fait que la couche B soit située "entre" les couches A et C ne signifie pas qu'elle se trouve obligatoirement en contact avec celles-ci ; en effet, une ou plusieurs couches intermédiaires peuvent être interposées entre la couche B d'une part et la couche A et/ou C d'autre part.

Comme couche intermédiaire, on peut notamment ajouter une couche répondant à la même définition que la couche A, B ou C décrites ci-dessus. Une variante particulièrement avantageuse de ce type est une structure à 4 couches de type A/C'/B/C, dans laquelle la couche C' répond à la définition de la couche C donnée ci-dessus. On peut également ajouter une ou plusieurs couches intermédiaires à base d'un ou plusieurs polymères thermoplastiques quelconques, mais que l'on choisit de préférence parmi les polymères du type polyoléfine-cétone (POK), copolymères d' oléfines cycliques (COC), poly-isobutylène (PIB), ou les polyesters élastomériques tels que les copolymères à blocs à base d'acide carboxylique de cycloalcane et de diols (PCCE,...). Dans certaines applications, il peut en outre être avantageux de prévoir une couche barrière, constituée par exemple d'un copolymère éthylène-alcool vinylique (EVOH) ou d'un polyamide (PA). Si une ou plusieurs couches intermédiaires sont ajoutées entre la couche A et la couche B, on préfère que leur épaisseur cumulée n'excède pas 30 % de l'épaisseur totale de la structure. Il en va de même pour une ou plusieurs éventuelles couches intermédiaires qui seraient ajoutées entre la couche C et la couche B. On peut également choisir comme couche intermédiaire, une couche contenant de la matière recyclée à base de structures selon la présente invention.

De même, une ou plusieurs couches extérieures à base de polymère(s) thermoplastique(s) peuvent être ajoutées du côté de la couche A et/ou de la couche C opposé à la couche B. Ainsi, par exemple, les structures suivantes peuvent répondre à la définition de l'invention: D/A/B/C; A/B/E/C; D/A/B/E/F/C; D/A/E/B/E/C/D/F. La ou les éventuelles couches extérieures sont à base d'un ou plusieurs polymères thermoplastiques quelconques, mais que l'on choisit de préférence parmi les homopolymères du propylène ainsi que parmi les polymères des types EVOH, POK, polyéthylène-téréphtalate, PET, polycarbonate, polyamide (en particulier PA 11 et/ou PA12) et copolymères d' oléofines cycliques (COC). D'excellents résultats ont été obtenus avec des structures comprenant une couche de POK ou de polyamide voisine de la couche A (mais pouvant cependant être séparée de cette dernière par une couche d'adhésif).

De bons résultats ont également été obtenus lorsque la couche superficielle du côté de la couche A contient du COC (éventuellement en mélange avec de SEBS et du VLDPE) et/ou du PP homopolymère mélangé à du SEBS. Cette couche superficielle peut selon le cas être la couche A elle-même ou une couche extérieure à celle-ci. Le choix d'une telle couche superficielle permet d'éviter l'adhérence des structures selon la présente invention entre elles et/ou à un éventuel matériau d'emballage, par exemple à base de polyoléfines (tels que les mélanges de PE et d'isobutène).

Si une ou plusieurs couches extérieures sont ajoutées à la structure du côté extérieur de la couche A (c'est-à-dire du côté opposé à la couche B), on préfère que leur épaisseur cumulée n'excède pas 25 % de l'épaisseur totale de la structure. Il en va de même pour une ou plusieurs éventuelles couches extérieures ajoutées à la structure du côté extérieur de la couche C.

Il va de soi que toutes les couches de la structure doivent être exemptes de polymères du chlorure de vinyle et de plastifiants de faible masse moléculaire.

Si nécessaire, une couche d'adhésif peut être interposée entre deux couches voisines. Tout adhésif connu peut être utilisé ; on utilise de préférence comme adhésifs une polyoléfine greffée ou modifiée par un anhydride, un acrylate, de l'acide acrylique, un composé glycidylique ou du monoxyde de carbone, ou encore un mélange d'un ou plusieurs tels composés avec des polymères usuels tel qu'une polyoléfine ou un polyester.

En outre, il est préférable que le module d'élasticité global de la structure n'excède pas 500 MPa, de préférence 350 MPa, de manière particulièrement préférée 200 MPa.

Bien que les structures selon l'invention puissent présenter une épaisseur quelconque, leur épaisseur est généralement faible, par exemple de l'ordre du millimètre pour les feuilles et les tubes, et de l'ordre de 100 à 300 µm pour les films, y compris les récipients constitués d'un ou de deux films soudés sur leur périphérie. Selon une variante avantageuse de l'invention, la structure présente une épaisseur n'excédant pas 400 µm, et plus préférentiellement 300 µm.

Les différentes caractéristiques décrites ci-dessus permettent d'obtenir des structures particulièrement transparentes. De préférence, leur rapport trouble / épaisseur est inférieur à 10 %/200 µm, et de manière particulièrement préférée inférieur à 6 %/200 µm. Le trouble (optique) ("haze") étant ici mesuré selon la norme ASTM D1003. Pour cette mesure, au cas où la structure serait grainée sur une ou deux de ses faces (c'est-à-dire présenterait une rugosité - paramètre Ra, mesuré selon la norme DIN 4768 - supérieure à 0,5 µm sur toute ou sur une partie de cette ou de ces faces), il serait nécessaire de mouiller la structure au moyen d'eau et de l'emprisonner entre deux plaques de verre parfaitement lisses et transparentes. Ce rapport n'a naturellement un sens que pour des structures minces, ayant une épaisseur n'excédant pas environ 500 µm. Selon une autre variante préférentielle concernant la transparence, la structure de l'invention présente de préférence une clarté (telle que définie par la norme ASTM D1003) d'au moins 95 %.

Un avantage particulièrement important des structures de l'invention est qu'elles sont capables de répondre aux exigences de la Pharmacopée européenne (European Pharmacopoeia, version 3.1.6., supplément 1999) et des normes américaines (USP 23, 1995 for Class VI plastics-121 °C).

D'autres avantages des structures de l'invention sont les suivants :
- faible température d'initiation pour le scellage (environ 125 °C), et large plage de scellage (au moins 15 °C) ;
- faible quantités de composés extractibles à l'hexane bouillant (au reflux) (moins de 5 % en poids, voire moins de 1 %) ;
- coefficient transmission de vapeur d'eau (norme ASTM F1249) inférieur à 4 g/m²/jour (à 23 °C) ;
- des sachets dont la paroi est une structure selon l'invention, ayant un volume d'un litre, remplis d'eau à température ambiante, résistent à une chute de 2 mètres sur une surface plane.

Au vu de ces propriétés remarquables, l'invention concerne également une structure multicouche à base de polymères thermoplastiques, substantiellement exempte de polymères du chlorure de vinyle et de plastifiants de faible masse moléculaire, comprenant au moins 3 couches, d'une épaisseur totale comprise entre 100 et 400 µm, ladite structure présentant simultanément :
- un module d'élasticité de moins de 350 MPa,
- une quantité de composés extractibles à l'hexane bouillant de moins de 5 %,
- un rapport trouble/épaisseur inférieur à 6 % / 200 µm.

Sur base des indications qui précèdent, la fabrication des structures de l'invention est à la portée de l'homme du métier. Ces structures peuvent notamment être fabriquées par coextrusion.

De préférence, en vue de réduire la cristallinité d'une polyoléfine à cristallinité contrôlable comprise dans la structure, le procédé de fabrication de la structure comprend au moins une étape d'étirage se déroulant à une température comprise entre la température de cristallisation (Tc) de la polyoléfine et Tf + 15 °C, Tf désignant sa température de fusion, une étape de relaxation se déroulant dans la même plage de température, et une étape de refroidissement brutal. Davantage de détails concernant un tel procédé de modification de la cristallinité sont fournis dans la demande de brevet EP 832730 (SOLVAY).

Dès lors, un autre objet de la présente invention concerne un procédé de fabrication d'une structure multicouche à base de polymères thermoplastiques, substantiellement exempte de polymères du chlorure de vinyle et de plastifiants de faible masse moléculaire, comprenant au moins 3 couches :
- une première couche (A) comprenant au moins 60 % en poids d'au moins une polyoléfine à cristallinité contrôlable, l'épaisseur de la première couche étant d'au moins 20 % de l'épaisseur totale de la structure, la première couche ayant un module d'élasticité inférieur à 350 MPa;
- une seconde couche (B), disposée entre la première (A) et la troisième (C) couche, comprenant au moins 40 % en poids d'au moins une polyoléfine à cristallinité contrôlable, la seconde couche ayant globalement une température de ramollissement inférieure à 121 °C ;
- une troisième couche (C) comprenant au moins 50 % en poids d'au moins une polyoléfine à cristallinité contrôlable, l'épaisseur de la troisième couche étant de 5 à 30 % de l'épaisseur totale de la structure, et la troisième couche ayant un module d'élasticité inférieur à celui de la première couche ;
dans laquelle la polyoléfine à cristallinité contrôlable est définie comme comprenant au moins 90 % de propylène et comme ayant une température de ramollissement inférieure à 121 °C, selon lequel les différentes couches de la structure sont coextrudées, puis sont soumises:
- à au moins une étape d'étirage d'au moins 100 % se déroulant à une température comprise entre la température de cristallisation maximale des polyoléfines à cristallinité contrôlable (Tc) et Tf + 15 C, Tf désignant leur température de fusion minimale, et
- à au moins une étape de relaxation à dimensions constantes durant au moins 10 s, se déroulant dans la même plage de température, puis
- à une étape de refroidissement brutal à une température inférieure à la température de cristallisation maximale Tc.

Les préférences indiquées ci-dessus en rapport avec la structure proprement dite s'appliquent également en ce qui concerne le procédé.

La température de cristallisation maximale des polyoléfines à cristallinité contrôlable est définie comme étant la plus élevée des températures de cristallisation des polyoléfines à cristallinité contrôlable présentes dans les couches A, B et C. Leur température de fusion minimale est définie comme étant la plus faible des températures de fusion des polyoléfines à cristallinité contrôlable présentes dans les couches A, B et C.
Les conditions de l'étirage sont avantageusement telles que le gradient de vitesse soit d'au moins 1/ (20.τ₀), et de préférence d'au moins 1/ (10.τ₀), où τ₀ désigne le temps moyen de relaxation tel que défini ci-dessus. L'étape d'étirage provoque un étirement monoaxial ou biaxial d'au moins 100 %, et de préférence d'au moins 300 %.

La relaxation s'effectue à dimensions constantes et en l'absence de toute contrainte extérieure. Elle s'effectue de préférence à la surface d'un cylindre maintenu à la température appropriée, avec lequel la structure entre en contact après son étirage.

Selon une variante avantageuse, plusieurs étapes de relaxation sont séparées par plusieurs étapes d'étirage, le facteur d'étirage et le temps de relaxation susmentionnés étant des valeurs totales. Cette variante peut se réaliser en utilisant plusieurs cylindres tournant à des vitesses différentes.

Le refroidissement brutal contribue à accroître la transparence et la souplesse de la structure. Il s'effectue de préférence à une température inférieure à T_{c} - 50 °C. Ce refroidissement peut notamment se réaliser en faisant passer la structure dans un bain d'eau thermostatisé ou sur un cylindre réfrigéré ; un simple refroidissement par de l'air n'est pas suffisamment efficace. Les procédés d'extrusion-soufflage conviennent dès lors mal.

Eventuellement, une ou chacune des faces de la structure de l'invention peut être grainée, c'est-à-dire munie d'un relief présentant une rugosité (Ra) de plus de 0,5 µm. En vue de grainer les deux faces de la structure, il est avantageux d'utiliser deux cylindres de grainage métalliques, de dureté élevée, grainant simultanément (et non séquentiellement) les deux faces de la structure, sur au moins une partie de leur surface (des "fenêtres" non grainées pouvant en effet être prévues sur l'une ou chacune des faces). Davantage de détails sur ce procédé sont fournis dans la demande de brevet EP 743163 (SOLVAY). Cette éventuelle étape de grainage intervient de préférence entre l'étape d'étirage et l'étape de relaxation.

Les structures décrites ci-dessus sont particulièrement avantageuses lorsqu'on les utilise dans le domaine médical, par exemple pour la réalisation de films, feuilles, tubes, récipients ou articles similaires destinés à entrer en contact avec des tissus biologiques, ou avec des fluides biologiques ou médicamenteux tels que du sang ou des solutés pour perfusion. En particulier, l'invention concerne également une structure telle que définie ci-dessous, se présentant sous la forme d'un tube, d'un film ou d'un récipient. Plus particulièrement, elle concerne également une poche souple obtenue à partir de deux films tels que décrits ci-dessus, ou encore à partir d'un film tubulaire, que l'on soude sur leur périphérie de manière à obtenir un récipient hermétique (muni toutefois de conduits appropriés permettant son remplissage et sa vidange).

La structure faisant l'objet de l'invention est particulièrement destinée à être utilisée de telle façon que ce soit la couche C qui entre en contact avec des tissus biologiques ou des fluides biologiques ou médicamenteux. Dès lors, l'invention concerne en particulier un tube ou un récipient réalisé au moyen de la structure susmentionnée, dont la couche C est dirigée vers l'intérieur. De préférence, la couche C constitue la couche superficielle intérieure dudit tube ou récipient.

La structure peut naturellement être avantageusement utilisée pour d'autres applications, par exemple pour l'emballage ou le transport de fluides quelconques, par exemple de produits alimentaires tels que des boissons, de la confiture, etc.

Dans le cas où la couche C présente globalement une température de fusion supérieure à 121 °C, mais comprend néanmoins au moins un polymère dont la température de fusion est inférieure à 121 °C, la structure selon la présente invention peut être soudée sur elle-même ou sur une autre structure ayant une couche externe de même composition que la couche C (la couche C jouant le rôle de couche de scellage), de manière à obtenir une soudure dont la force de pelage est reproductible.

Dès lors, la présente invention concerne également un article obtenu par soudure d'une structure avec une telle couche C sur elle-même ou sur une autre structure ayant une couche externe de même composition que cette couche C.

En outre, la soudure d'une telle couche C peut être adaptée de manière à être très solide (permanente) ou au contraire, aisément pelable. On appelle soudure aisément pelable selon cette variante préférée de la présente invention, une soudure dont la force de pelage déterminée selon la norme ASTM F88 est inférieure à 3000 N/m, de préférence inférieure à 2000 N/m. Cette force de pelage est avantageusement supérieure à 300 N/m, voire supérieure à 600 N/m. Les structures selon cette variante avantageuse présentent en outre l'avantage que la force de pelage des soudures est peu variable d'une pièce soudée à l'autre, et ce surtout après stérilisation de ces pièces à 121°C durant au moins 10 min. Il peut s'avérer intéressant d'utiliser les structures selon cette variante de la présente invention pour fabriquer des récipients hermétiques (c.à.d. comprenant une ou des soudures ou autres fixations permanentes qui les rendent étanches et leur permettent donc de contenir des fluides) séparés en au moins deux compartiments par une soudure aisément pelable.

Dès lors, la présente invention concerne également un récipient hermétique obtenu à partir d'un article tel que décrit ci-avant, comprenant au moins une soudure permanente et séparé en au moins deux compartiments par une soudure aisément pelable.

Un tel récipient peut par exemple être utilisé pour contenir des fluides qui doivent être stockés et/ou stérilisés séparément et mélangés avant emploi par rupture de la soudure aisément pelable. Des exemples de tels fluides sont le glucose et certaines vitamines et/ou acides aminés ; et les solution de bicarbonate de sodium et certaines solutions acides.

Dès lors, la présente invention concerne également l'utilisation de tels récipients hermétiques pour le stockage de fluides à conserver et/ou stériliser séparément et à mélanger juste avant emploi par rupture de la soudure aisément pelable.

Pour la réalisation de tels récipients hermétiques à plusieurs compartiments, les températures de soudure sont choisies en fonction des matériaux de la structure qui les constitue. Avantageusement, la température choisie pour la soudure aisément pelable est inférieure à celle de la soudure permanente d'au moins 5°C.

Dès lors, la présente invention concerne également un procédé de fabrication d'un tel récipient hermétique où la soudure aisément pelable est réalisée à une température inférieure à celle de la soudure permanente d'au moins 5°C.

On choisit avantageusement la température de la soudure permanente au moins égale à 125°C, voire 130°C ; cette température ne dépasse de préférence pas 160°C, ou mieux, 150°C. La température de la soudure pelable est avantageusement d'au moins 95°C, de préférence d'au moins 115°C ; elle ne dépasse de préférence pas 140°C, ou mieux, 130°C.

### Exemples

Les exemples suivants illustrent l'invention de façon non limitative.

### Exemple 1

On a fabriqué par coextrusion un film de 200 µm d'épaisseur, comprenant trois couches (A/B/C) :
La couche A, épaisse de 60 µm, était constituée d'un copolymère du propylène comprenant environ 5 % d'éthylène (ELTEX® P KS 409, de SOLVAY). Bien que la température de ramollissement (point Vicat) (Tv) de ce copolymère mentionnée par le fabricant soit d'environ 123 °C, le procédé de fabrication utilisé dans cet exemple (comprenant notamment une étape d'étirage d'un facteur 4 se déroulant à 120 °C, une étape de relaxation durant 18 s se déroulant approximativement à la même température, et une étape de refroidissement brutal à 15 °C) a permis que le film présente une faible cristallinité et une température de ramollissement réelle d'environ 110 °C.
La couche B, épaisse de 100 µm, était constituée de 50 % du même copolymère (ELTEX® P KS 409) et de 50 % d'un copolymère de l'éthylène et de 1-octène (contenant environ 14 % d'octène) (DEX EXACT® 8201).
La couche C, épaisse de 40 µm, était constituée d'un mélange de 70 % du même copolymère du propylène que dans la couche A (ELTEX P KS409), de 22,5 % de SEBS (KRATON® G1657), de 5 % de copolymère éthylène-acétate de vinyle (EVA) et de 2,5 % de copolymère éthylène - acrylate de méthyle (EMA) (ces deux derniers polymères ayant des températures de fusion d'environ 70 °C).

La structure ainsi obtenue présentait un trouble de 3,1 % et un module d'élasticité de 170 MPa. En outre, des poches de 220 x 130 mm réalisées à partir de cette structure, remplies d'un litre d'eau à 4 °C, résistaient à une chute de 2 m sur une surface plane.

### Exemple comparatif 1

On a fabriqué par coextrusion un film de 200 µm d'épaisseur, comprenant trois couches (A/B/C) :
La couche A, épaisse de 15 µm, était constituée d'un hompolymère du propylène (ELTEX® P HV 424, de Solvay). La température de ramollissement (point Vicat) (Tv) de ce copolymère mentionnée par le fabricant est d'environ 156°C.
La couche B, épaisse de 135 µm, était constituée de 50 % du même copolymère (ELTEX® P KS 409) que la couche B de l'exemple 1 et de 50 % d'un copolymère de l'éthylène et de 1-octène (contenant environ 14 % d'octène) (DEX EXACT® 8201).
La couche C, épaisse de 50 µm, était constituée d'un mélange d'un copolymère du propylène (ELTEX P KS409) à 77,5% et de SEBS (KRATON® G1657) à 22.5%.

La structure ainsi obtenue présentait un trouble de 12,1 % et un module d'élasticité de 440 MPa. En outre, des poches de 220 x 130 mm réalisées à partir de cette structure, remplies d'un litre d'eau à 4 °C, ne résistaient pas à une chute de 1 m sur une surface plane.

### Exemple 2

On a fabriqué par coextrusion un film de 200 µm d'épaisseur, comprenant quatre couches (A/B1/B2/C), en utilisant le procédé décrit dans l'exemple 1 :
La couche A, épaisse de 74 µm, a la même composition que la couche A de l'exemple 1. La couche B1, épaisse de 14 µm, a la même composition que la couche C de l'exemple 1. La couche B2, épaisse de 90 µm, a la même composition que la couche B de l'exemple 1. La couche C, épaisse de 22 µm, a la même composition que la couche C de l'exemple 1.

La structure ainsi obtenue présentait un trouble de 5,6 % et un module d'élasticité de 155 MPa. En outre, des poches de 220 x 130 mm réalisées à partir de cette structure, remplies d'un litre d'eau à 4 °C, résistaient à une chute de 2 m sur une surface plane.

### Exemple 3

On a fabriqué par coextrusion un film de 200 µm d'épaisseur, comprenant quatre couches (A/B1/B2/C), en utilisant le procédé décrit dans l'exemple 1 :
La couche A, épaisse de 90 µm, a la même composition que la couche A de l'exemple 1. La couche B1, épaisse de 20 µm, a la même composition que la couche C de l'exemple 1. La couche B2, épaisse de 70 µm, était constituée de 45 % du même copolymère (ELTEX P KS409), de 40 % du copolymère propylène-éthylène HUNTSMAN W204 (contenant environ 6 % d'éthylène, présentant une très large distribution des masses moléculaires ; Tf=148 °C, Tv=80 °C), et de 15 % de copolymère à bloc SEBS (KRATON G1657). La couche C, épaisse de 20 µm, a la même composition que la couche C de l'exemple 1.

La structure ainsi obtenue présentait un trouble de 4,2 %, un module d'élasticité de 154 MPa, un rapport contrainte à la rupture / contrainte au seuil d'écoulement de 2,8, un coefficient de transmission de la vapeur d'eau de 3 g/m²/jour, et une température de fragilisation de-19 °C. En outre, des poches de 220 x 130 mm réalisées à partir de cette structure, remplies d'un litre d'eau à 4 °C, résistaient à une chute de 2 m sur une surface plane.

### Exemple 4

On a fabriqué par coextrusion un film de 205 µm d'épaisseur, comprenant quatre couches (A/B 1/B2/C), en utilisant le procédé décrit dans l'exemple 1 :
La couche A, épaisse de 85 µm, a la même composition que la couche A de l'exemple 1. La couche B1, épaisse de 20 µm, a la même composition que la couche C de l'exemple 1. La couche B2, épaisse de 80 µm, est constituée d'un mélange de 56 % du même copolymère du propylène que dans la couche A (ELTEX P KS409), de 11 % de copolymère éthylène-butène TAFMER® A4085 (Tv = 54 °C), de 11 % de copolymère propylène-butène TAFMER XR107L (Tv = 91 °C), de 11 % de polybutène TAFMER BL 4000 (Tv = 116 °C ; Tf = 125 °C), et de 11 % de copolymère à blocs SEBS (KRATON G 1657). La couche C, épaisse de 20 µm, a la même composition que la couche C de l'exemple 1.

La structure ainsi obtenue présentait un trouble de 2,8 %, un module d'élasticité de 165 MPa, un rapport contrainte à la rupture / contrainte au seuil d'écoulement de 2,3, et une température de fragilisation de -15 °C.

### Exemple 5

On a fabriqué une structure comme exposé dans l'exemple 3, à la différence près que la couche A était constituée de 60 % de copolymère propylène-éthylène contenant environ 3 % d'éthylène (ELTEX P KL104 de Solvay) (Tv =114 °C) et de 40 % de polybutène-1 (TAFMER BL 4000).

La structure ainsi obtenue présentait un trouble de 2,3 %, un module d'élasticité de 155 MPa, et permettait de fabriquer des poches qui, remplies d'un litre d'eau, résistaient à une chute de 2 m.

### Exemple 6

On a fabriqué une structure comme exposé dans l'exemple 5, à la différence près que la couche A était constituée de 75 % de copolymère ELTEX P KL104 et de 25 % de copolymère propylène-butène-1 contenant plus de 10 % de butène (TAFMER XR107L).

La structure ainsi obtenue présentait un trouble de 2,8 %, un module d'élasticité de 150 MPa, et permettait de fabriquer des poches qui, remplies d'un litre d'eau, résistaient à une chute de 2 m.

### Exemple 7

On a fabriqué une structure comme exposé dans l'exemple 5, à la différence près que la couche A était constituée de 65 % de copolymère ELTEX P KL104, de 25 % de l'homopolymère propylène ELTEX P HL402 et de 10 % de copolymère à blocs SEBS (KRATON G 1657).

La structure ainsi obtenue présentait un trouble de 3,7 %, un module d'élasticité de 190 MPa, et permettait de fabriquer des poches qui, remplies d'un litre d'eau, résistaient à une chute de 2 m.

Par rapport à la structure obtenue dans l'exemple 5, cette structure présente une moindre adhérence à une emballage à base de polyoléfines.

### Exemple 8

On a fabriqué par coextrusion un film de 200 µm d'épaisseur, comprenant quatre couches (A/B1/B2/C). La couche A, épaisse de 50 µm, était constituée 85 % en poids d'un mélange de polyoléfines à cristallinité contrôlable et de 15 % de copolymère à bloc SEBS (KRATON G1657). Le mélange de polyoléfines à cristallinité contrôlable est constitué d'un copolymère de propylène-éthylène à 4 % d'éthylène (KFC2004, de Borealis) et d'un copolymère de propylène-éthylène comprenant environ 3% d'éthylène (ELTEX® P KL104, de SOLVAY). Le procédé de fabrication utilisé dans cet exemple (comprenant notamment une étape d'étirage d'un facteur 4 se déroulant à 120 °C, une étape de relaxation durant 18 s se déroulant approximativement à la même température, et une étape de refroidissement brutal à 15°C) a permis que le film présente une faible cristallinité et une température de ramollissement réelle d'environ 115°C. La couche B1, épaisse de 60 µm, était constituée de 100 % du copolymère propylène-éthylène HUNTSMAN W203. La couche B2, épaisse de 70 µm, était constituée de 50 % du copolymère ELTEX® P KL 104 et de 50 % du copolymère propylène-éthylène HUNTSMAN W209 (contenant environ 5 % d'éthylène; Tf=120 °C, Tv<23°C). La couche C, épaisse de 20 µm, était constituée d'un mélange de 75 % du copolymère ELTEX® P KL104, de 22 % de SEBS (KRATON® G1657), de 2 % de copolymère éthylène-acétate de vinyle (EVA) et de 1 % d'un copolymère éthylène - acrylate de méthyle (EMA) (ces deux derniers polymères ayant des températures de fusion d'environ 70 °C).

La structure ainsi obtenue présentait un trouble de 3.9 % et un module d'élasticité de 175 MPa. En outre, des poches de 220 x 130 mm réalisées à partir de cette structure, remplies d'un litre d'eau à 4 °C, résistaient à une chute de 2 m sur une surface plane.

### Exemple 9

Même structure que dans l'exemple 8, sauf que la couche C, épaisse de 20 µm, était constituée d'un mélange de 78.5 % d'un copolymère du propylène-éthylène (ELTEX® P KL104), de 20 % de SEBS (KRATON® G1657), de 1 % d'un copolymère éthylène-acétate de vinyle (EVA) et de 0.5 % d'un copolymère éthylène - acrylate de méthyle (EMA) (ces deux derniers polymères ayant des températures de fusion d'environ 70 °C).

Des essais de soudure ont été réalisés avec les structures obtenues dans les exemples 8 et 9. Les résultats de ces essais figurent ci-après :

**Tableau 1 : soudures avec les structures selon l'exemple 8: force de pelage en N/m après sterilisation à 121 °C durant 30 min (resultats obtenus selon ASTM F88)**

| Durée soudure (s) | 0.2 | 0.4 | 0.6 | 0.8 | 1.0 | 1.2 | 1.4 | 1.6 |
|---|---|---|---|---|---|---|---|---|
| T° (°C) soudure | | | | | | | | |
| 145° C | 3128 | | | | | | | |
| 136° C | 3012 | 3343 | | | | | | |
| 134 °C | | | | 3288 | | | | |
| 132 °C | 970 | 1180 | 1280 | 1400 | | | | |
| 130°C | | 831 | 1042 | 1295 | | | | 1498 |
| 128 °C | | | 1086 | | | 1104 | | |
| 126 °C | | 887 | | 940 | | 1111 | | |
| 124 °C | | 1124 | 1151 | | 1092 | 1101 | 946 | |
| 122°C | | 951 | 1092 | | | | | 1189 |
| 120°C | | | | | 1351 | | | 1140 |
| 118 °C | | | | 1218 | | | | |

**Tableau 2 : Force de pelage (en N/m et selon ASTM F88) d'une soudure obtenue à 125°C et en 0.6 s**

| | Détail des mesures | Valeur moyenne | Détail des mesures | Valeur moyenne |
|---|---|---|---|---|
| Structure selon | Exemple 8 | Exemple 8 | Exemple 12 | Exemple 12 |
| Avant stérilisation | 425 | 495 | 311 | 308 |
| | 435 | | 285 | |
| | 557 | | 340 | |
| | 543 | | 356 | |
| | 515 | | 248 | |
| Stérilisation à 121 °C durant 10 min | 1042 | 970 | 624 | 604 |
| | 898 | | 584 | |
| | | | | |
| Stérilisation à 121 °C durant 30 min | 1104 | 1226 | 705 | 677 |
| | 1235 | | 735 | |
| | 1274 | | 692 | |
| | 1346 | | 612 | |
| | 1171 | | 642 | |
| Stérilisation à 121°C durant 60 min | 901 | 981 | 643 | 680 |
| | 1060 | | 718 | |
| | | | | |
| Stérilisation à 125 °C durant 30 min | 1513 | 1533 | 940 | 927 |
| | 1390 | | 882 | |
| | 1611 | | 960 | |
| | 1688 | | 1002 | |
| | 1462 | | 852 | |

## Revendications

1. Structure multicouche à base de polymères thermoplastiques, substantiellement exempte de polymères du chlorure de vinyle et de plastifiants de masse moléculaire Mw inférieure à 1000, comprenant au moins 3 couches:
- une première couche (A) comprenant au moins 60 % en poids d'au moins une polyoléfine à cristallinité contrôlable, l'épaisseur de la première couche étant d'au moins 20 % de l'épaisseur totale de la structure, la première couche ayant un module d'élasticité inférieur à 350 MPa;
- une seconde couche (B), disposée entre la première (A) et la troisième (C) couche, comprenant au moins 40 % en poids d'au moins une polyoléfine à cristallinité contrôlable, la seconde couche ayant globalement une température de ramollissement inférieure à 121 °C;
- une troisième couche (C) comprenant au moins 50 % en poids d'au moins une polyoléfine à cristallinité contrôlable, l'épaisseur de la troisième couche étant de 5 à 30 % de l'épaisseur totale de la structure, et la troisième couche ayant un module d'élasticité inférieur à celui de la première couche et présentant globalement une température de fusion supérieure à 121 °C tout en comprenant un polymère ayant une température de fusion inférieure à 121°C dans laquelle la polyoléfine à cristallinité contrôlable est définie comme comprenant au moins 90 % de propylène et comme ayant une température de ramollissement inférieure à 121°C.

2. Structure selon la revendication 1, dans laquelle la polyoléfine à cristallinité contrôlable est un copolymère.

3. Structure selon l'une des revendications précédentes, dans laquelle la polyoléfine à cristallinité contrôlable est un copolymère comprenant au moins 90 % en poids de propylène ainsi que moins de 10 % d'un ou plusieurs autres comonomères choisis dans le groupe constitué d'éthylène, de butène, ainsi que des alcènes en C5 à C10.

4. Structure selon l'une des revendications précédentes, dans laquelle la première couche (A) présente globalement une température de fusion supérieure à 121°C.

5. Structure selon l'une des revendications précédentes, dans laquelle le ou les polymères constitutifs de la couche A sont exclusivement choisis parmi le groupe constitué des polyoléfines à cristallinité contrôlable et des polyoléfines peu cristallines ou amorphes.

6. Structure selon l'une quelconque des revendications précédentes, comprenant une ou plusieurs couches extérieures à base de polymère(s) thermoplastique(s) du côté de la couche A et/ou de la couche C opposé à la couche B.

7. Structure selon l'une des revendications précédentes, dont la couche superficielle du côté de la couche A comprend du COC et/ou du PP homopolymère mélangé à du SEBS.

8. Structure selon la revendication 6, dont la couche superficielle du côté de la couche A comprend du COC en mélange avec du SEBS et du VLDPE.

9. Procédé de fabrication d'une structure multicouche à base de polymères thermoplastiques, substantiellement exemple de polymères du chlorure de vinyle et de plastifiants de masse moléculaire Mw inférieure à 1000, comprenant au moins 3 couches:
- une première couche (A) comprenant au moins 60 % en poids d'au moins une polyoléfine à cristallinité contrôlable, l'épaisseur de la première couche étant d'au moins 20 % de l'épaisseur totale de la structure, la première couche ayant un module d'élasticité inférieur à 350 MPa;
- une seconde couche (B), disposée entre la première (A) et la troisième (C) couche, comprenant au moins 40 % en poids d'au moins une polyoléfine à cristatillinité contrôlable, la seconde couche ayant globalement une température de ramollissement inférieure à 121 °C;
- une troisième couche (C) comprenant au moins 50 % en poids d'au moins une polyoléfine à cristallinité contrôlable, l'épaisseur de la troisième couche étant de 5 à 30 % de l'épaisseur totale de la structure, et la troisième couche ayant un module d'élasticité inférieur à celui de la première couche; dans laquelle la polyoléfine à cristallinité contrôlable est définie comme comprenant au moins 90 % propylène et comme ayant une température de ramollissement inférieure à 121 °C.
selon lequel les différentes couches de la structure sont coextrudées, puis sont soumises:
- à au moins une étape d'étirage d'au moins 100 % se déroulant à une température comprise entre la température de cristallisation maximale des polyoléfines à cristallinité contrôlable (Tc) et Tf + 15 °C, Tf désignant leur température de fusion minimale, et
- à au moins une étape de relaxation à dimensions constantes durant au moins 10 s, se déroulant dans la même plage de température, puis
- à une étape de refroidissement brutal à une température inférieure à la température de cristallisation maximale Tc.

10. Article se présentant sous la forme d'un tube, d'un film ou d'un récipient obtenu à partir d'une structure selon l'une des revendications 1 à 8.

11. Article obtenu par soudure de la structure selon la revendication 1 sur elle-même ou sur une autre structure ayant une couche externe de même composition que la couche (C).

12. Récipient hermétique obtenu à partir d'un article selon la revendication 11, comprenant au moins une soudure permanente et séparé en au moins deux compartiments par une soudure aisément pelable.

13. Utilisation du récipient selon la revendication 12, pour le stockage de fluides à conserver et/ou stériliser séparément et à mélanger juste avant emploi par rupture de la soudure aisément pelable.

14. Procédé de fabrication d'un récipient selon la revendication 12, **caractérisée en ce que** la soudure aisément pelable est réalisée à une température inférieure à celle de la soudure permanente d'au moins 5°C.

15. Procédé selon la revendication 14, **caractérisée en ce que** la soudure permanente est réalisée à une température comprise entre 125 et 160°C, et la soudure aisément pelable, à une température comprise entre 95 et 140°C.

## Claims

1. Multilayer structure based on thermoplastic polymers, substantially devoid of vinyl chloride polymers and of plasticizers of molecular mass M_{w} less than 1000, comprising at least 3 layers:
- a first layer (A) comprising at least 60% by weight of at least one polyolefin of controllable crystallinity, the thickness of the first layer being at least 20% of the total thickness of the structure, the first layer having a modulus of elasticity of less than 350 MPa;
- a second layer (B), positioned between the first layer (A) and the third layer (C), comprising at least 40% by weight of at least one polyolefin of controllable crystallinity, the second layer having an overall softening temperature of less than 121 °C;
- a third layer (C) comprising at least 50% by weight of at least one polyolefin of controllable crystallinity, the thickness of the third layer being from 5 to 30% of the total thickness of the structure and the third layer having a modulus of elasticity which is less than that of the first layer, and exhibiting an overall melting temperature of greater than 121 °C, besides comprising a polymer having a melting temperature of less than 121 °C wherein the polyolefin of controllable crystallinity is defined as comprising at least 90% of propylene and as having a softening temperature of less than 121 °C.

2. Structure according to claim 1, in which the polyolefin of controllable crystallinity is a copolymer.

3. Structure according to one of the preceding claims, in which the polyolefin of controllable crystallinity is a copolymer comprising at least 90% by weight of propylene and less than 10% of one or more other comonomers chosen from the group consisting of ethylene, butene as well as C₅ to C₁₀ alkenes.

4. Structure according to one of the preceding claims, in which the first layer (A), taken as a whole, exhibits a melting temperature of greater than 121 °C.

5. Structure according to one of the preceding claims, in which the constituent polymer(s) of the layer A are chosen exclusively from the group consisting of polyolefins of controllable crystallinity and of not very crystalline or amorphous polyolefins.

6. Structure according to one of the preceding claims, comprising one or more outer layers based on thermoplastic polymer(s) on the side of the layer A and/or of the layer C opposite the layer B.

7. Structure according to one of the preceding claims, the surface layer of which on the side of the layer A comprises COC and/or PP homopolymer mixed with SEBS.

8. Structure according to Claim 6, the surface layer of which on the side of the layer A comprises COC as a mixture with SEBS and VLDPE.

9. Process for the manufacture of a multilayer structure based on thermoplastic polymers which is substantially devoid of vinyl chloride polymers and of plasticizers of molecular mass M_{w} less than 1000, comprising at least 3 layers:
- a first layer (A) comprising at least 60% by weight of at least one polyolefin of controllable crystallinity, the thickness of the first layer being at least 20% of the total thickness of the structure, the first layer having a modulus of elasticity of less than 350 MPa;
- a second layer (B), positioned between the first layer (A) and the third layer (C), comprising at least 40% by weight of at least one polyolefin of controllable crystallinity, the second layer having an overall softening temperature of less than 121 °C;
- a third layer (C) comprising at least 50% by weight of at least one polyolefin of controllable crystallinity, the thickness of the third layer being from 5 to 30% of the total thickness of the structure and the third layer having a modulus of elasticity which is less than that of the first layer;
wherein the polyolefin of controllable crystallinity is defined as comprising at least 90% of propylene and as having a softening temperature of less than 121 °C
according to which the various layers of the structure are coextruded and are then subjected
- to at least one stage of drawing by at least 100%, which takes place at a temperature between the maximum crystallization temperature of the polyolefins of controllable crystallinity (Tc) and Tm+15 °C, Tm denoting their minimum melting temperature, and
- to at least one stage of relaxation at constant dimensions lasting at least 10 s, which takes place in the same temperature range, then
- to a stage of sudden cooling to a temperature of less than the maximum crystallization temperature Tc.

10. Article in the form of a tube, of a film or of a container obtained from a structure according to one of claims 1 to 8.

11. Article obtained by welding the structure according to claim 1 to itself or to another structure having an outer layer with the same composition as the layer (C).

12. Hermetic container obtained from an article according to claim 11 which comprises at least one permanent weld and which is separated into at least two compartments by an easily peelable weld.

13. Use of the container according to claim 12 for the storage of fluids to be retained and/or sterilized separately and to be mixed immediately before use by breaking the easily peelable weld.

14. Process for the manufacture of a container according to claim 12, wherein the easily peelable weld is realized at a temperature lower than that for the formation of the permanent weld by at least 5 °C.

15. Process according to Claim 14, wherein the permanent weld is carried out at a temperature of between 125 and 160 °C and the easily peelable weld is carried out at a temperature of between 95 and 140 °C.

## Patentansprüche

1. Mehrschichtige Struktur auf Basis thermoplastischer Polymere, die im wesentlichen frei von Polyvinylchlorid und Weichmachern mit einer Molmasse M_{w} unterhalb 1000 ist, umfassend mindestens drei Lagen:
- eine erste Lage (A) umfassend wenigstens 60 Gew.-% mindestens eines Polyolefins mit kontrollierbarer Kristallinität, die Dicke der ersten Lage beträgt mindestens 20 % der Gesamtdicke der Struktur, die erste Lage hat ein Elastizitätsmodul von weniger als 350 MPa;
- eine zweite Lage (B), angeordnet zwischen der ersten (A) und der dritten (C) Lage, umfassend wenigstens 40 Gew.-% mindestens eines Polyolefins mit kontrollierbarer Kristallinität, die zweite Lage hat insgesamt eine Erweichungstemperatur unter 121 °C;
- eine dritte Lage (C) umfassend wenigstens 50 Gew.-% mindestens eines Polyolefins mit kontrollierbarer Kristallinität, die Dicke der dritten Lage beträgt von 5 bis 30 % der Gesamtdicke der Struktur, und die dritte Lage hat ein kleineres Elastizitätsmodul als die erste Lage und weist insgesamt eine Schmelztemperatur über 121 °C auf, obwohl ein Polymer mit einer Schmelztemperatur unter 121 °C enthalten ist;
wobei das Polyolefin mit kontrollierbarer Kristallinität mindestens 90 Gew.-% Propylen umfasst und eine Erweichungstemperatur unter 121 °C aufweist.

2. Struktur gemäß Anspruch 1, worin das Polyolefin mit kontrollierbarer Kristallinität ein Copolymer ist.

3. Struktur gemäß einem der vorhergehenden Ansprüche, worin das Polyolefin mit kontrollierbarer Kristallinität ein Copolymer ist, umfassend mindestens 90 Gew.-% Propylen sowie weniger als 10 Gew.-% eines oder mehrerer Comonomere ausgewählt unter Ethylen, Buten, sowie C₅ bis C₁₀ Alkenen.

4. Struktur gemäß einem der vorhergehenden Ansprüche, worin die erste Lage (A) insgesamt eine Schmelztemperatur über 121 °C aufweist.

5. Struktur gemäß einem der vorhergehenden Ansprüche, worin das oder die die Lage A bildenden Polymere ausschließlich aus der Gruppe bestehend aus Polyolefinen mit kontrollierbarer Kristallinität und wenig kristallinen oder amorphen Polyolefinen ausgewählt sind.

6. Struktur gemäß einem der vorhergehenden Ansprüche, umfassend eine oder mehrere weitere, äußere Lagen auf Basis von thermoplastischem(n) Polymer(en) auf der der Lage B gegenüberliegenden Seite der Lage A und/oder der Lage C.

7. Struktur gemäß einem der vorhergehenden Ansprüche, worin die Oberflächenlage auf der Seite der Lage A COC und/oder PP-Homopolymer gemischt mit SEBS umfasst.

8. Struktur gemäß Anspruch 6, worin die Oberflächenlage auf der Seite der Lage A COC gemischt mit SEBS und VLDPE umfasst.

9. Verfahren zur Herstellung einer mehrschichtigen Struktur auf Basis thermoplastischer Polymere, die im wesentlichen frei von Polyvinylchlorid und Weichmachern mit einer Molmasse M_{w} unterhalb 1000 ist, umfassend mindestens drei Lagen:
- eine erste Lage (A) umfassend wenigstens 60 Gew.-% mindestens eines Polyolefins mit kontrollierbarer Kristallinität, die Dicke der ersten Lage beträgt mindestens 20 % der Gesamtdicke der Struktur, die erste Lage hat ein Elastizitätsmodul von weniger als 350 MPa;
- eine zweite Lage (B), angeordnet zwischen der ersten (A) und der dritten (C) Lage, umfassend wenigstens 40 Gew.-% mindestens eines Polyolefins mit kontrollierbarer Kristallinität, die zweite Lage hat insgesamt eine Erweichungstemperatur unter 121 °C;
- eine dritte Lage (C) umfassend wenigstens 50 Gew.-% mindestens eines Polyolefins mit kontrollierbarer Kristallinität, die Dicke der dritten Lage beträgt von 5 bis 30 % der Gesamtdicke der Struktur, und die dritte Lage hat ein kleineres Elastizitätsmodul als die erste Lage;
wobei das Polyolefin mit kontrollierbarer Kristallinität mindestens 90 Gew.-% Propylen umfasst und eine Erweichungstemperatur unter 121 °C aufweist nach welchem die verschiedenen Lagen der Struktur coextrudiert sind, und anschließend:
- mindestens einem Reckschritt um mindestens 100 %, der bei einer Temperatur zwischen der maximalen Kristallisationstemperatur des Polyolefins mit kontrollierbarer Kristallinität (Tc) und Tf + 15 °C abläuft, wobei Tf deren minimale Schmelztemperatur bezeichnet, und
- mindestens einem wenigstens 10 Sekunden dauernden Relaxationsschritt bei konstanter Ausdehnung, der in demselben Temperaturbereich abläuft, danach
- einem Schritt sprunghafter Abkühlung auf eine Temperatur unter der maximalen Kristallisationstemperatur Tc unterworfen werden.

10. Artikel in Form eines Schlauches, einer Folie oder eines Behälters, erhalten aus einer Struktur gemäß einem der Ansprüche 1 bis 8.

11. Artikel erhalten durch Schweißen einer Struktur gemäß Anspruch 1 mit sich selber oder mit einer anderen Struktur, die eine äußere Lage mit derselben Zusammensetzung wie Lage (C) aufweist.

12. Hermetischer Behälter erhalten aus einem Artikel gemäß Anspruch 11, umfassend zumindest eine permanente Schweißnaht und durch eine leicht peelbare Schweißnaht aufgeteilt in zumindest zwei Kammern.

13. Verwendung eines Behälter gemäß Anspruch 12 zur Lagerung von Flüssigkeiten, die separat aufzubewahren und/oder zu sterilisieren sind und direkt vor der Anwendung durch Trennen der leicht peelbaren Schweißnaht vermischt werden.

14. Verfahren zur Herstellung eines Behälters gemäß Anspruch 12, **dadurch gekennzeichnet, dass** die leicht peelbare Schweißnaht bei einer Temperatur realisiert wird, die um mindestens 5 °C unter der für die permanente Schweißnaht liegt.

15. Verfahren gemäß Anspruch 14, **dadurch gekennzeichnet, dass** die permanente Schweißnaht bei einer Temperatur zwischen 125 und 160 °C realisiert wird und die leicht peelbare Schweißnaht bei einer Temperatur zwischen 95 und 140 °C.
